(19) Europäisches Patentamt
European Patent Office
Office européen des brevets

(11) Publication number: **0 155 934**
**B1**

(12) # EUROPEAN PATENT SPECIFICATION

(45) Date of publication of patent specification: **06.12.89**

(51) Int. Cl.$^4$: **A 61 K 31/56**

(21) Application number: **83902717.4**

(22) Date of filing: **29.07.83**

(86) International application number: **PCT/US83/01156**

(87) International publication number: **WO 85/00519 14.02.85 Gazette 85/04**

(54) **Pharmaceutical compositions for treating androgen-related disorders in humans.**

(43) Date of publication of application: **02.10.85 Bulletin 85/40**

(45) Publication of the grant of the patent: **06.12.89 Bulletin 89/49**

(84) Designated Contracting States: **DE FR GB**

(56) References cited:
**US-A-4 055 641**

**STEROIDS, vol. 38, no. 2, August 1981, pages 121-141, Holden Day, San Francisco, US; V. PETROW et al.: "Prostatic cancer. I. 6-methylene-4-pregnen-3-ones as irreversible inhibitors of rat prostatic delta4-3 ketosteroid 5alpha-reductase"**

**J. Steroid Biochem. 1980 vol 13, 1261-4**

**J. Endocr. 95(1982) 311-3**

**J.Pharm. Pharmacol. 36(1984) 352-3**

**The Prostate 6(1985) 129-143**

**The file contains technical information submitted after the application was filed and not included in this specification**

(73) Proprietor: **Duke University**
**Office of Patent Administration 614 Chapel Drive Annex**
**Durham North Carolina 27706 (US)**

(72) Inventor: **PETROW, Vladimir**
**Route 5 Box 198**
**Chapel Hill, NC 27514 (US)**
Inventor: **LACK, Leon**
**2936 Wellcome Drive**
**Durham, NC 27705 (US)**

(74) Representative: **Giambrocono, Alfonso, Dr. Ing.**
**Ing. A. Giambrocono & C. S.r.l. Via Rosolino Pilo 19/B**
**I-20129 Milano (IT)**

(56) References cited:
**The Prostate 9(1986) 343-361**

**Biochem. Cell Biol 65 626-34(1987)**

**Endocrinol Japan, 34, 279-289(1987)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 155 934 B1**

**Description**

Technical Field

This invention relates to pharmaceutical compositions useful for the treatment of androgen-related disorders in humans.

Background Art

Considerable experimental evidence exists supporting the conclusions that the 5α-reduced metabolite of testosterone (II), 5α-dihydrotestosterone (III)

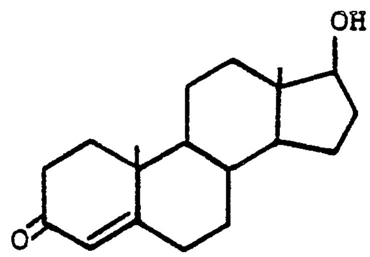

(II)

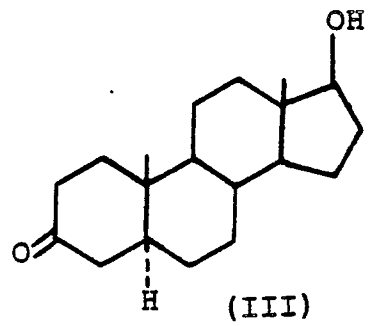

(III)

is the active form of the androgenic hormone responsible for eliciting somatic androgenic effects, and that testosterone (II) is, *de facto,* a prohormone [cf. for example, Gloyna, R. E. and Wilson, J. D., J. Clin. Endocrinol. 29:970 (1969); Mainwaring, W. I. P., Mangan, F. R., Wilce, P. A. and Melroy, E. G. P., Advances in Experimental Medicine and Biology, 36:197 (1973); Liao, S., International Review of Cytology, 41:87 (1975)]. It is consequently generally accepted that androgen-related disorders stem from excessive production of dihydrotestosterone in the body. Such androgen-related disorders include acne, oily skin, seborrhea, androgenic alopecia, hirsutism, androgen-dependent prostatic cancer, prostatic hypertrophy and virilism.

It follows that treatment, or palliative treatment in the case of prostatic carcinoma, of these disorders may be effected by inhibiting the conversion of (II) into (III).

The conversion of testosterone (II) into dihydrotestosterone (III) in the body is effected by the NADPH-dependent enzyme 5α-reductase. Treatment of androgen-related disorders may thus be achieved by inhibiting the enzyme 5α-reductase. This fact is well-documented in the literature (cf. for example, U.S.P. 3,917,829; U.S.P. 4,088,760). Progesterone appears to be a preferred substrate for the enzyme (cf. for example, Voight, W., Fernandez, E. P. and Hsia, S. L., J. Biol. Chem. 245:5594 (1970)), and is well-known to be a reversible and competitive inhibitor of the enzyme. It is therefore not surprising that progesterone has been used to counteract excessive dihydrotestosterone production. Thus topical administration of a 0.5% solution of progesterone in aqueous ethanol caused an important decrease in sebum secretion in 45/53 males with acne [cf. Vermorken, A. J. M. and Jouben, J. J. G., Drug. Intel, Clin. Pharm., 12:151—157 (1978)]. A pro-drug form of progesterone is claimed in Bodor, N. S. and Sloan, K. B., U.S.P. 4,213,978/1980, as useful in the treatment of acne and seborrhea. Progesterone strongly inhibits the enzyme in cell-culture preparations of human prostate thereby inhibiting growth of the tissue [Sandberg, A., U.I.C.C. Technical Report Series 48:165 (1979), see also Massa, R. and Martini, L., Gynec. Invest. 2:253 (1971/2)]. Inhibition of the conversion of testosterone to dihydrotestosterone by progesterone in preparations of human benign prostatic hypertropic tissue has been reported by Tau, S. Y., Antonpillai, I. and Pearson Murphy, B. E. [J. Clin. Endocrinol. Metab. 39:936 (1974)]. However, the value of progesterone as an inhibitor of 5α-reductase, and hence as a therapeutic agent in the treatment of androgen-related disorders, is limited by the following

(i) It is a competitive (reversible) inhibitor of the enzyme. It is now widely recognised that an irreversible inhibitor offers a distinct advantage over a reversible inhibitor in that it can induce prolonged inactivation of the enzyme and combat the effects of physiological dilution [cf. for example, Shaw, E., in Enzyme Inhibitors as Drugs, Ed. Sandler, M., MacMillan Press, p. 25, 1980];

(ii) It undergoes metabolism in the body to androstenedione and other androgenic metabolites and is thus unsuitable for systemic administration.

A need therefore exists for progesterone derivatives which are irreversible inhibitors of the enzyme 5α-reductase.

Disclosure of the Invention

It is therefore an object of the invention to provide pharmaceutical compositions for the treatment of androgen-related disorders in humans which comprises 5α-testosterone decreasing amounts of a compound of the formula (I)

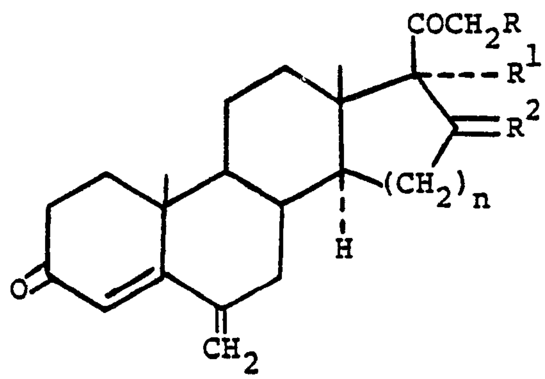

(I)

wherein

R is H or F

R' is selected from the group consisting of —H; straight or branched chain lower alkyl; hydroxyl; —$OCOR^3$ and O—($C_1$—$C_6$ alkyl); wherein $R^3$ is —H, $C_1$—$C_{10}$ straight or branched chain alkyl group, phenyl, phenyl alkylene having straight or branched chain $C_1$—$C_6$ alkylene, $C_5$—$C_{10}$ cycloalkyl or $C_6$—$C_{10}$ cycloalkylalkylene;

$R^2$ is $H_2$, methylene, ethylidene, α-$CH_3$(H), β-$CH_3$(H), α(OH)H, or the acetonide derived from the 16α,17α-dihydroxy derivative, and n is 1 or 2, together with an inert pharmaceutically acceptable carrier.

Brief Description of the Drawings

Figure 1 shows the time course of inactivation of 5α-reductase following incubation of the enzyme with NADPH and 17α-acetoxy-6-methyleneprogesterone; see Example 2.

Figure 2 demonstrates that the inactivation of the enzyme 5α-reductase follows saturation kinetics, since the plot of the rate constants (as T 1/2's) versus 1/[Inhibitor] is linear: see Example 2.

Best Mode for Carrying Out the Invention

As used herein the term androgen-related disorder is intended to mean any disease or condition resulting from overproduction of dihydrotestosterone in the body including acne, oily skin, seborrhea, androgenic alopecia, hirsutism, virilism, androgen-dependent prostatic carcinoma and benign prostatic hypertrophy.

For a more detailed description of these conditions, see for example Harrison's Principles of Internal Medicine, 9th Edition, McGraw Hill, 1980, Volume 1, pp. 227-229 (hirsutism, virilism), volume 1, pages 242—243 (acne), volume 2, pages 1771—1772 (cancer of the prostate).

It is the object of this invention to provide pharmaceutical preparations of the steroids of formula (I) which can be administered to a patient suffering from an androgen-related disorder; this novel method of treatment offers considerable advantages over prior art, for example over estrogen therapy, in that it is free from deleterious side effects such as estrogenization.

The compounds used in the invention have the formula (I):

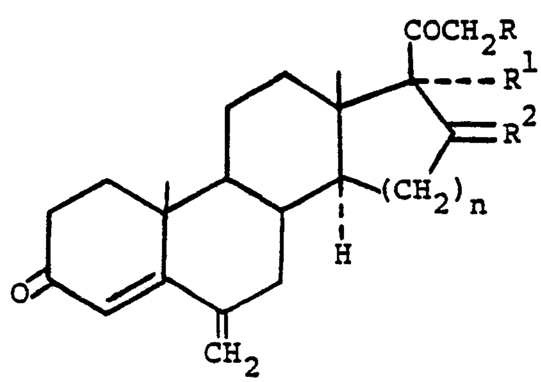

(I)

wherein

R is H or F;

R' is H; lower alkyl containing from 1 to 6 carbon atoms, which may be straight or branched chain such as for example methyl, ethyl, n-propyl, butyl, isobutyl and the like; hydroxyl; $OCOR^3$ wherein $R^3$ may be H, an alkyl moiety containing from 1 to 10 carbon atoms and may be straight or branched chain, such as for example methyl, ethyl, n-propyl, isopropyl, n-butyl, sec-butyl, tert-buty, n-pentyl, neopentyl, pivalyl, hexyl, heptyl and octyl; phenyl; phenylalkyl (Ph-alkyl-) wherein the alkyl moiety (which may also be referred to as an alkylene moiety) has from 1 to 6 carbon atoms and can be straight or branched chain; cycloalkyl wherein

the cycloalkyl moiety has from 5 to 10 carbon atoms such as cyclopentyl-, cyclohexyl-, cycloheptyl, cyclooctyl- and their alkylene derivatives containing from 6 to 16 carbon atoms such as cyclopentylmethylene $C_5H_9CH_2$—; O-lower alkyl, wherein the alkyl group has from 1 to 6 carbon atoms and may be straight or branched chain such for example as methyl-, ethyl-, propyl-, iso-propyl-, iso-pentyl, butyl, isobutyl, pentyl;

$R^2$ is $H_2$, methylene, ethylidene, α-Me(H), β-Me(H), α-(OH)H, and the acetonide derived from the 16α, 17α-dihydroxy derivative;

n is 1 or 2.

Preferred embodiments of this invention include the following derivatives of 6-Methylene-progesterone:

17α-acetoxy-
17α-acetoxy-D-homo-
17α-acetoxy-21-fluoro
17α-acetoxy-21-fluoro-D-homo
17α-caproyloxy-
17α-caproyloxy-D-homo
17α-caproyloxy-21-fluoro-
17α-caproyloxy-21-fluoro-d-homo; the 16α-methyl-, 16β-methyl- and 16-methylene and ethylidene derivatives of the above (when n = 1),
17α-methyl-
17α-methyl-D-homo
17α-methyl-21-fluoro-
17α-methyl-21-fluoro-D-homo; the 17α-ethyl analogues of the above and their 16α- and 16β-methyl-derivatives (when n = 1),
17α-methoxy-
17α-methoxy-D-homo
17α-methyl-21-fluoro-
17α-methyl-21-fluoro-D-homo-; the 17α-ethyl analogues of the above and their 16α- and 16β-methyl-derivatives (when n = 1),
17α-methoxy-
17α-methoxy-D-homo-
17α-methoxy-21-fluoro-
17α-methoxy-21-fluoro-D-homo-
17α-ethoxy-
17α-ethoxy-D-homo-
17α-ethoxy-21-fluoro-
17α-ethoxy-21-fluoro-D-homo-; the 16α-methyl, 16β-methyl and 16-methylene and ethylidene derivatives of the above (when n = 1)
acetonide from 16α,17α-dihydroxy derivative (when n = 1)
acetonide from the 21-fluoro-16α,17α-dihydroxy derivative (when n = 1) and the D-homo analogs of the above
6-methylene progesterone and its
21-fluoro-
16α-methyl-
21-fluoro-16α-methyl-
16β-methyl
21-fluoro-16β-methyl and
D-homo analogues of the above.

Most of the compounds claimed in this invention are already known in the art. Those that are not known can be readily prepared from the known and appropriate progesterone derivatives by the Vilsmeier or analogous processes as reported, for example, in the following publications:

D. Burn *et al,* Tetrahedron 20:597 (1964)
F. Schneider *et al,* Helv. Chim. Acta 56:2396 (1973)
M. Muller *et al,* Helv. Chim. Acta 63:1857 (1980)
D. Burn *et al,* Tetrahedron 21:569 (1965)
D. N. Kirk and V. Petrow, U.S.P. 3,112,305
F. B. Colton, U.S.P. 2,980,711
The Upjohn Co. B.P. 1,271,207.

V. Petrow, *et al,* in Steroids, Vol. 38, No. 2 (08/1981), pages 121—141, described preliminary *in vitro* experiments to assess the potential activity of the compounds of the present invention as irreversible inhibitors of rat prostatic $\Delta^4$-3-ketosteroid 5α-reductase. No mention is made in said paper of the actual possibility of utilizing the compounds in human therapy, particularly in topical formulations, nor is any useful information given about useful dosages and toxicity.

The compounds employed in the present invention can be administered in various manners to achieve the desired dihydrotestosterone-decreasing effect. The compounds can be administered alone or in the form of pharmaceutical preparations to the patient being treated orally, parenterally or topically.

Topical administration is preferred for acne and seborrhea. The amount of compound administered will vary with the severity of the condition being treated. For oral and parenteral administration the daily dose will generally be from 0.1 to 50 mg/Kg and preferably from 1 to 30 mg/Kg. Unit dosages for oral or parenteral administration may contain, for example, from 5 to 500 mg of the active ingredient.

For topical administration effective amounts of the compounds of general formula (I) on a percent basis may vary from 0.001% to 5% and preferably from 0.005% to 1%. For topical administration the formulated active ingredient, that is a compound of general formula I, can be applied directly to the site requiring treatment or can be applied to the oral or nasal mucosa. Applicator sticks carrying the formulation can be, for example, in the form of a solution, suspension, emulsion, gel or cream of either the oil-in-water or water-in-oil type, ointment, paste, jelly, paint or powder. Suitable bases for the topical preparation may be of any conventional type such as oleaginous bases, for example, olive oil, cottonseed oil, petrolatum, white petrolatum, mineral oils, silicones, such as dimethylpolysiloxane, or methylphenylpolysiloxane, lanolins, polyethyleneglycol, glyceryl monostearate, methylcellulose and hydroxymethylcellulose. The topical formulation may contain pharmaceutically acceptable surfactants, wetting agents, dispersing agents, emulsifiers, penetrants, emollients, detergents, hardeners, preservatives, fillers, antioxidants, perfumes, cooling agents, such as methnol, soothing agents, such as camphor, or coloring agents, such as zinc oxide. Aerosol preparations of a solution, suspension or emulsion containing the active ingredient in the form of a finely ground powder can also be employed for topical administration. The aerosol container together with a gaseous or liquified propellant, for example, dichlorofluoromethane, dichlorodifluoromethane with dichlorodifluoroethane, carbon dioxide, nitrogen, or propane with the usual adjuvant such as cosolvent and wetting agents as may be necessary or desirable. The compounds may also be administered in a non-pressurized form such as in a nebulizer or atomizer.

For oral administration the compounds can be formulated into solid or liquid preparations, such as capsules, pills, tablets, troches, powders, solutions, suspensions or emulsions. The compounds can be applied in the form of an aerosol containing finely divided particles of the active ingredient. The solid unit dosage forms can be a capsule which can be of the ordinary gelatin type containing a compound of general formula I and a carrier, for example, lubricants and inert filler such as lactose, sucrose, and corn starch. In another embodiment the compounds of the general formula I can be tableted with conventional tablet bases such as lactose, sucrose and corn starch in combination with binders such as acacia, corn starch or gelatin, disintegrating agents such as potato starch or aliginic acids and a lubricant such as stearic acid or magensium stearate.

For parenteral administration the compounds may be administered as injectable dosages of a solution or suspension of the compound in a physiologically acceptable diluent with a pharmaceutical carrier which can be a sterile liquid such as water-in-oil with or without the addition of a surfactant and other pharmaceutically acceptable adjuvants. Illustrative of oils which can be employed in these preparations are those of petroleum, animal, vegetable or synthetic origin, for example, peanut oil, soybean oil and mineral oil. In general, water, saline, aqueous dextrose and related sugar solutions, ethanols and glycols, such as propylene glycol or polyethylene glycol are preferred liquid carriers, particularly for injectable solutions.

The compounds can be administered in the form of a depot injection or inplant preparation which can be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers and synthetic silicones. For example, Silastic®, silicone rubber manufactured by the Dow-Corning Corporation.

The compounds of general Formula I in treating acne and oily skin conditions may be used in combination with other anti-acne preparations, antiseptics, anti-infective agents, keratolytic agents, for example, benzoic acid, resorcinol or salicyclic acid, and comedolytic agents, such as, retinoic acid or agents having a retinoic acid-like action, corticoids or other antiinflammatory agents, thioglycolates, ethyl lactate or benzoyl peroxide.

In using the products of this invention, topical administration is preferred for acne and seborrhea. The remaining conditions are preferably treated by systemic administration. In treating benign prostatic hypertrophy and prostatic carcinoma, improved results are obtained by administering the products of the invention concurrently with megestrol acetate, chlormadinone acetate, medrogestone or cyproterone acetate at therapeutic dose levels.

Having now generally described this invention, the same will be better understood by reference to certain specific examples.

Biological Results

Example 1

The compounds of the present invention represent an important advance over progestrone and derivatives thereof since they are irreversible inhibitors of the enzyme 5α-reductase. Employing the assay of R. J. Moore and J. D. Wilson [Methods in Enzymology, Vol. XXXVI, Academic Press, N.Y., Ed. W. O'Malley and G. Hardman, p. 466—474 (1975)], it is found that 6-methyleneprogesterone and 17α-acetoxy-6-methyleneprogesterone, for example are equipotent with progesterone as inhibitors of the enzyme. On

preincubating the enzyme with 17α-acetoxy-6-methyleneprogestrone and NADPH, diluting tenfold and assaying for 5α-reductase activity, it is surprisingly found, however, that 75% of the enzyme activity is lost. Similar preincubation of the enzyme with progesterone, in striking contrast, does not result in enzyme inactivation. These results are tabulated below, in Tables 1 and 2.

TABLE 1

Effect of Preincubation of Enzyme with 17-Acetoxy-6-methylene-4-pregnen-3,20-dione and NADPH on 5α-Reductase Activity

| | Preincubation conditions Time: 15 min | | Enzymic Assay conditions Time: 45 min | | | Picomol Testosterone reduced/mg protein in 45 min + SEM |
|---|---|---|---|---|---|---|
| | Inhibitor M | NADPH | Inhibitor M | Testosterone | NADPH | |
| A | $5x10^{-7}$ | $6x10^{-5}$ | $5x10^{-8}$ | $5x10^{-8}$ | $5x10^{-4}$ | $0.71 \pm 0.018$ n = 6 |
| B | O | $6x10^{-5}$ | $5x10^{-8}$ | $5x10^{-8}$ | $5x10^{-4}$ | $3.0 \pm 0.26$ n = 6 |
| C | O | O | $5x10^{-8}$ | $5x10^{-8}$ | $5x10^{-4}$ | $2.83 \pm 0.09$ n = 6 |
| D | $5x10^{-7}$ | O | $5x1-^{-8}$ | $5x10^{-8}$ | $5x10^{-4}$ | $2.63 \pm 0.18$ n = 6 |
| E | No preincubation | | O | $5x10^{-8}$ | $5x10^{-4}$ | $4.36 \pm 0.24$ n = 4 |
| F | No preincubation | | $5x10^{-8}$ | $5x10^{-8}$ | $5x10^{-4}$ | $3.14 \pm 0.20$ n = 4 |

n = number of experiments

TABLE 2

Effect of Preincubation of Enzyme with Progesterone and NADPH on 5α-Reductase Activity

| | Preincubation conditions Time: 15 min | | Conditions during enzymic assay 15 min | | | Picomol Testosterone Reduced/mg protein in 45 min |
|---|---|---|---|---|---|---|
| | Progesterone | NADPH | Progesterone | NADPH | Testosterone | |
| 1 | O | $6x10^{-5}$ | $5x10^{-8}$ | $5x10^{-4}$ | $5x10^{-8}$ | 3.91 |
| | O | $6x10^{-5}$ | $5x10^{-8}$ | $5x10^{-4}$ | $5x10^{-8}$ | 3.41 |
| 2 | $5x10^{-7}$ | $6x10^{-5}$ | $5x10^{-8}$ | $5x10^{-4}$ | $5x10^{-8}$ | 3.91 |
| | $5x10^{-7}$ | $6x10^{-5}$ | $5x10^{-8}$ | $5x10^{-4}$ | $5x10^{-8}$ | 3.79 |
| 3 | No preincubation | | O | $5x10^{-4}$ | $5x10^{-8}$ | 5.80 |
| | No preincubation | | O | $5x10^{-4}$ | $5x10^{-8}$ | 5.42 |
| 4 | No preincubation | | $5x10^{-8}$ | $5x10^{-4}$ | $5x10^{-8}$ | 3.66 |
| | No preincubation | | $5x10^{-8}$ | $5x10^{-4}$ | $5x10^{-8}$ | 5.54 |

These observations reveal that 17α-acetoxy-6-methyleneprogesterone, in striking contrast to progesterone, combines with the enzyme in the presence of the co-factor NADPH in an irreversible manner, whilst progesterone inactivation of the enzyme is competitive and reversible.

Example 2

The time course of inactivation of 5α-reductase following incubation of the enzyme with NADPH and 17α-acetoxy-6-methyleneprogesterone is shown in Fig. 1.

This time course of inactivation of the enzyme can be seen to follow pseudo first-order kinetics, which is in accord with the postulate that the inhibition invoked by such preincubation exposure is irreversible. When these rate constants are plotted (as the t 1/2's) against the reciprocal of the inhibitor concentrations, a straight line i obtained with a positive intercept on the y-axis, indicating a saturation phenomenon (Fig. 2). These data are in accord with the conclusion that the interaction of the inhibitor with the enzyme shows two phases. The first is a reversible combination of the enzyme and inhibitor with a Ki of $1.25 \times 10^{-6}$M. The enzyme-inhibitor complex then undergoes irreversible combination rendering the enzyme inactive. The rate constant for this step ($k_{cat}$) is $4.8 \times 10^{-3}\ s^{-1}$.

Formulations

Following are illustrative topical pharmaceutical formulations which may be employed in practicing the present invention:

Example 3

Solution

| | |
|---|---|
| 17α-Acetoxy-6-methyleneprogesterone | 0.85 g |
| Alcohol | 78.9 ml |
| Isopropyl Myristate | 5.0 g |
| Polyethylene Glycol 400 | 10.0 g |
| Purified Water qs ad | 100 ml |

Combine the alcohol, isoproyl myristate and polyethylene glycol 400 and dissolve the drug substance therein. Add sufficient purified water to give 100 ml.

Example 4

A Gel

| | |
|---|---|
| 17α-Acetoxy-6-methyleneprogesterone | 0.85 g |
| Alcohol | 78.9 ml |
| Isopropyl Myristate | 5.0 g |
| Polyethylene Glycol 400 | 10.0 g |
| Carbopol® 940 (Carboxypolymethylene) | 0.75g |
| Triethylamine | qs |
| Purified Water qs ad | 85 g |

Disperse the Carbopol 940 in the isopropyl myristate. To 38 ml of alcohol add 7 ml of purified water and the polyethylene glycol 400 and mix. Combine the two phases and mix until well dispersed. Add sufficient triethylamine to give a neutral pH. Dissolve the drug substance in the balance of the alcohol and mix well into the batch. Add and mix sufficiently purified water to provide 85 g of finished product.

Example 5

Applicator Stick

| | |
|---|---|
| 17α-Acetoxy-6-methyleneprogesterone | 0.85 g |
| Absolute Alcohol | 75 ml |
| Polyethylene Glycol 400 | 10.0 g |

| | |
|---|---|
| Isopropyl Myristate | 5.0 g |
| Stearic Acid | 4.3 g |
| Sodium Hydroxide | 0.55 g |
| Purified Water qs ad | 85 g |

Combine the absolute alcohol, polyethylene glycol 400 and isopropyl myristate and dissolve the drug substance therein. Add the stearic acid and heat the mixture to about 65°C. Dissolve the sodium hydroxide in a small amount of water, add and mix . Add sufficient water to provide 85 g of finished product. Pour into suitable molds and allow to solidify.

Example 6
Aerosol Foam

| | |
|---|---|
| 17α-Acetoxy-6-methyleneprogesterone | 1.0 g |
| Propylene Glycol | 96.0 g |
| Emulsifying Wax NF XIV | 3.0 g |
| Dichlorodifluoromethane: cryflourane (20:80) | 6.9 g |

Dissolve the drug substance in the propylene glycol. Add the smulsifying wax and heat to approximately 70°C. Stir while cooling to room temperature. Charge a suitable aerosol unit with this concentrate and 6.9 g of dichlorodifluoromethane : cryofluorane (20:80).

Example 7
Topical Cream, Vanishing, o/w

| | |
|---|---|
| 17α-Acetoxy-6-methyleneprogesterone | 1 |
| Stearic Acid | 15 |
| Sorbitan Monostearate | 2 |
| Polyoxyethylene Sorbitan Monostearate | 2.3 |
| Propylene Glycol | 5 |
| Methylparaben | 0.025% |
| Propylparaben | 0.015% |
| Purified Water | qs |

Example 8
Buccal or Sublingual Tablet

| | |
|---|---|
| 17α-Acetoxy-6-methyleneprogesterone | 1% |
| Calcium Stearate | 1% |
| Calcium Saccharin | 0.02% |
| Granular Mannitol | qs |

Mix and compress on a suitable tablet machine to a weight of 0.115 g/tablet.

Example 9
Powder

| | |
|---|---|
| 17α-Acetoxy-6-methyleneprogesterone micronized | 1 |

| | |
|---|---|
| Silicone dioxide, anhydrous | 0.5 |
| Corn starch, lactose, fine powder aa | qs |

Example 10
Oleaginous Ointment

| | |
|---|---|
| 17α-Acetoxy-6-methyleneprogesterone | 1 |
| White wax | 5 |
| White petrolatum qs | 100 |

Example 11
Absorption Ointment Base

| | |
|---|---|
| 17α-Acetoxy-6-methyleneprogesterone | 1 |
| Cholesterol | 3 |
| Stearyl alcohol | 3 |
| White wax | 8 |
| White petrolatum qs | 100 |

Example 12
Water Soluble Ointment Base

| | |
|---|---|
| 17α-Acetoxy-6-methyleneprogesterone | 1 |
| Polyethylene glycol 4000 | 40 |
| Polyethylene glycol 400 qs | 100 |

Example 13
Paste

| | |
|---|---|
| 17α-Acetoxy-6-methyleneprogesterone | 1 |
| Starch | 25 |
| Zinc oxide | 25 |
| White petrolatum qs | 100 |

Example 14
Aersol Foam

| | |
|---|---|
| 17α-Acetoxy-6-methyleneprogesterone | 1 |
| Emulsifying wax | 3 |
| Stearic acid | 1 |
| Stearyl alcohol | 1 |
| Diglycol stearate | 2 |
| Propylene glycol | 92 |

The following are illustrative pharmaceutical formulations suitable for oral or parenteral administration which may be employed in practicing the present invention:

Example 15

| | |
|---|---|
| Tablet | For 15,000 |
| 17α-Acetoxy-6-methyleneprogesterone | 75 g |
| Lactose | 1.216 Kg |
| Corn Starch | 0.3 Kg |

Mix the active ingredient, the lactose and corn starch uniformly. Granulate with 10% starch paste. Dry to a moisture content of about 2.5%. Screen through a No. 12 mesh screen. Add and mix the following:

| | |
|---|---|
| Magnesium Stearate | 0.015 Kg |
| Corn starch qs ad | 1.725 Kg |

Compress on a suitable tablet machine to a weight to 0.115 g/tablet.

Example 16
Soft Gelatin Capsule

| | |
|---|---|
| 17α-Acetoxy-6-methyleneprogesterone | 0.25 Kg |
| Polysorbate 80 | 0.25 Kg |
| Corn Oil qs ad | 25.0 kg |

Mix and fil into 50,000 soft gelatin capsules.

Example 17
IM Depot Injection

Each 1 ml contains the following:

| | |
|---|---|
| 17α-Acetoxy-6-methyleneprogesterone | 5.0 mg |
| Anydroux Cholorobutanol | 5.0 mg |
| Aluminium Monostearate | 50.0 mg |
| Peanut Oil qs ad | 1.0 ml |

Dissolve or disperse the ingredients in the peanut oil.

Example 18
Depot-Implant

| | |
|---|---|
| 17α-Acetoxy-6-methyleneprogesterone | 5.0 mg |
| Anhydrous Chlorobutanol | 5.0 mg |
| Aluminum Monostearate | 50.0 mg |
| Peanut Oil qs ad | 1.0 ml |

Dissolve or disperse the ingredients in the peanut oil.

Example 18
Depot-Implant

| | |
|---|---|
| 17α-Acetoxy-6-methyleneprogesterone | 5.0 mg |
| Dimethylsiloxane | 240.0 mg |
| Catlyst qs | |

Disperse the drug substance in the fluid dimethylsiloxane. Add the catalyst and cast into a suitable monolytic structure.

Alternatively, the drug substance may be enclosed by a precast polydimethylsiloxane envelope.

Alternatively, the drug substance may be dispersed in a suitable amount of hydroxyethyl acrylate subsequently polymerized and cross-linked by the addition of ethylenedimethacrylate, and an oxidizing agent, to yield a 3-dimensional ethylene glycomethacrylate mouldable gel (Hydron®).

Example 19

IM Injections

A. Oil type

| | |
|---|---|
| 17α-Acetoxy-6-methyleneprogesterone | 25.0 mg |
| BHA, BHT aa | 0.01% w/v |
| Peanut Oil or Sesame Oil qs | 1.0 ml |

B. Suspension Type

| | |
|---|---|
| 17α-Acetoxy-6-methyleneprogesterone | 25.0 mg |
| Sodium Carboxymethylcellulose | 0.5% w/v |
| Sodium Bisulfite | 0.02% w/v |
| Water for Injection, qs | 1.0 ml |

Example 20

Buccal or Sublingual Tablet

| | |
|---|---|
| 17α-Acetoxy-6-methyleneprogesterone | 1% |
| Calcium Stearate | 1% |
| Calcium Saccharin | 0.02% |
| Granular Mannitol | qs |

Mix and compress on a suitable tablet machine to a weight of 0.115 g/tablet.

The following formulations are illustrative of phramaceutical preprations for topical application comprising a compound of general Formula I in combination with a keratolytic agent.

Example 21

| Aerosol Foam | % w/w |
|---|---|
| 17α-Acetoxy-6-methyleneprogesterone | 0.85 g |
| Resorcinol | 0.85 g |
| Alcohol | 78.9 ml |
| Isopropyl myristate | 5.0 g |
| Polyethylene glycol 400 | 10.0 g |
| Carbopol 940 (carboxypolymethylene) | 0.75 g |
| Triethylamine | qs |
| Purified water qs ad | |

Disperse the Carbopol 940 in the isopropyl myristate. To 38 ml of alcohol add 7 ml of purified water and the polyethylene glycol 400 and mix. Combine the two phases and mix until well dispersed. Add sufficient triethylamine to give a neutral pH. Dissolve the drug substance and the resorcinol in the balance

of the alcohol and mix well in to the batch. Add and mix sufficient purified water to provide 85 g of finished product.

## Claims

1. A pharmaceutical composition for treating androgen-related disorders in humans, which comprises 5α-dihydrotestosterone decreasing amounts of a compound of formula (I):

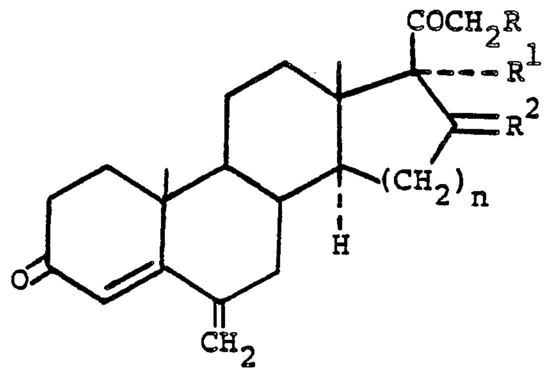

(I)

wherein

R is H or F;

$R^1$ is selected from —H; straight of branched chain lower alkyl; hydroxyl; —$OCOR^3$; and O—($C_1$—$C_6$ alkyl); wherein $R^3$ is —H, $C_1$—$C_{10}$ straight or branched chain alkyl group, phenyl, phenyl alkylene having straight or branched chain $C_1$—$C_6$ alkylene; $C_5$—$C_{10}$ cycloalkyl or $C_6$—$C_{10}$ cycloalkyl alkylene;

$R^2$ is $H_2$, methylene, ethylidene, α-$CH_3$(H), β-$CH_3$(H), α-(OH)H or the acetonide derived from the 16α, 17α-dihydroxy derivative, and

n is 1 or 2, together with an inert pharmaceutically acceptable carrier.

2. The pharmaceutical composition of Claim 1 wherein the androgen-related disorder is selected from acne, seborrhea, and androgenic alopecia.

3. The pharmaceutical composition of Claim 2 wherein the compound is administered as a topical preparation containing from 0.001% to 5% of the compound.

4. The pharmaceutical composition of Claim 1 wherein the androgen-related disorder is selected from oily skin, hirsutism, benign prostatic hypertrophy and androgen dependent prostatic adenocarcinoma.

5. The pharmaceutical composition of Claim 4 wherein the compound is administered in an amount of from 0.1 to 50 mg/Kg.

6. The pharmaceutical composition of Claim 4 where the compound is administered parenterally in an amount of from 0.1 to 50 mg/Kg.

7. The pharmaceutical composition of Claim 4 wherein said disorder is androgen dependent prostatic adenocarcinoma and the compound is administered together with a compound selected from megestrol acetate, medrogestone and cyproterone acetate.

8. The pharmaceutical composition of Claim 1 wherein $R = R^1 = H$, $R^2$ is $=H_2$ and $n = 1$.

9. The pharmaceutical composition of Claim 1 wherein $R = H$, $R^1 = OAc$, $R^2$ is $=H_2$ and $n = 1$.

10. The pharmaceutical composition of Claim 1 wherein $R = H$, $R^1 = OAc$, $R^2$ is $=CH_2$ and $n = 1$.

11. The pharmaceutical composition of Claim 1 wherein $R = H$, $R^1 = OAc$, $R^2$ is α-Me(H) and $n = 1$.

12. The pharmaceutical composition of Claim 1 wherein $R = H$, $R^1 = OAc$, $R^2$ is β-Me(H) and $n = 1$.

13. The pharmaceutical composition of Claim 1 wherein $R = H$, $R^1 = OAc$, $R^2$ is $=H_2$ and $n = 2$.

14. The pharmaceutical composition of Claim 1 wherein $R = R^1 = H$, $R^2$ is $=H_2$ and $n = 2$.

15. A pharmaceutical composition for topical application to the skin of a patient suffering from an androgen-related disorder which comprises 5α-dihydrotestosterone level decreasing amount of a compound of the formula:

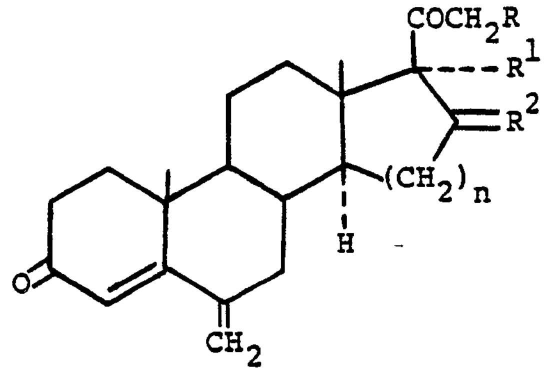

(I)

wherein

R is H or F;

$R^1$ is selected from —H; straight or branched chain lower alkyl; hydroxyl; —$OCOR^3$ and O—($C_1$—$C_6$ alkyl); wherein $R^3$ is —H, $C_1$—$C_{10}$ straight or branched chain alkyl group, phenyl, phenyl alkylene having straight or branched chain $C_1$—$C_{10}$ straight or branched chain alkyl group, phenyl, phenyl alkylene having straight or branched chain $C_1$—$C_6$ alkylene; $C_5$—$C_{10}$ cycloalkyl or $C_6$—$C_{10}$ cycloalkyl alkylene;

$R^2$ is $H_2$, methylene, ethylidene, α-$CH_3$(H), β-$CH_3$(H), α-OH(H) or the acetonide derived from the 16α,17α-dihydroxy derivative, and

n is 1 or 2;

together with an inert topical pharmaceutical carrier.

16. The composition of Claim 15 wherein said carrier is selected from oleaginous bases, silicones, lanolines, polyethylene glycol, glyceryl monostearate, methylcellulose and hydroxymethylcellulose.

**Patentansprüche**

1. Pharmazeutische Zusammensetzung für die Behandlung androgenbedingter Störungen bei Menschen, dadurch gekennzeichnet das sie aus einer 5α-Dihydrotestosteron herabsetzenden Menge einer Verbindung der Formel (I):

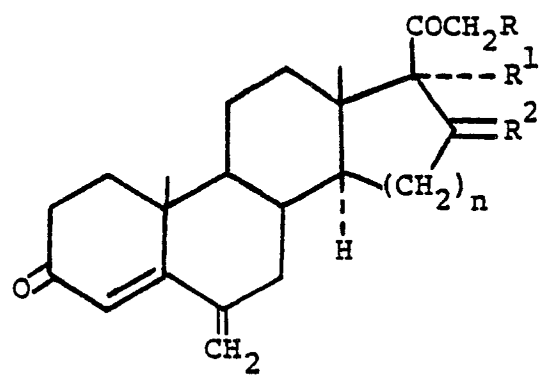

(I)

worin

R für H oder F steht;

$R^1$ aus —H; gerad- oder verzweigtkettigem niedrig Alkyl; Hydroxyl; —$OCOR^3$; und O—($C_1$—$C_6$-Alkyl); worin $R^3$ für H, eine $C_1$—$C_{10}$ gerad- oder verzweigtkettige Alkylgruppe, Phenyl, Phenylalkylen mit gerad- oder verzweigtkettigem $C_1$—$C_6$ Alkylen; $C_5$—$C_{10}$ Cycloalkyl oder $C_6$—$C_{10}$-Cycloalkylalkylen steht, ausgewählt ist;

$R^2$ für $H_2$, Methylen, Ethyliden, α-$CH_3CH$), β-$CH_3$(H), α-OH(H) oder für das vom 16α,17α-Dihydroxyderivat abgeleitete Acetonid steht und

n 1 oder 2 bedeutet, zusammen mit einem inerten pharmazeutisch zulässigen Träger besteht.

2. Pharmazeutische Zusammensetzung nach Anspruch 1, dadurch gekennzeichnet, daß die androgenbezogene Störung aus Akne, Seborrhoe und androgene Alopecie ausgewählt ist.

3. Pharmazeutische Zusammensetzung nach Anspruch 2, dadurch gekennzeichnet, daß die Verbindung als topisches Präparat enthaltend 0,001 bis 5% der Verbindung verabreicht wird.

4. Pharmazeutische Zusammensetzung nach Anspruch 1, dadurch gekennzeichnet, daß die androgene Störung aus fettige Haut, Hirsutismus, gutartige Prostatahypertrophie und androgenbedingtes Prostatacarcinom ausgewählt ist.

5. Pharmazeutische Zusammensetzung nach Anspruch 4, dadurch gekennzeichnet, daß die Verbindung oral in einer Menge von 0,1 bis 50 mg/kg verabreicht wird.

6. Pharmazeutische Zusammensetzung nach Anspruch 4, dadurch gekennzeichnet, daß die Verbindung parenteral in einer Menge von 0,1 bis 50 mg/kg verabreicht wird.

7. Pharmazeutische Zusammensetzung nach Anspruch 4, dadurch gekennzeichnet, daß die Störung androgenbedingtes Prostatacarcinom ist und die Verbindung zusammen mit einer Verbindung ausgewählt aus Megestrolacetat, Medrogeston und Cyproteronacetat ausgewählt ist.

8. Pharmazeutische Zusammensetzung nach Anspruch 1, dadurch gekennzeichnet, daß $R = R^1 = H$, $R^2 = H_2$ und $n = 1$.

9. Pharmazeutische Zusammensetzung nach Anspruch 1, dadurch gekennzeichnet, daß $R = H$, $R^1 = OAc$, $R^2 = H_2$ und $n = 1$.

10. Pharmazeutische Zusammensetzung nach Anspruch 1, dadurch gekennzeichnet, daß $R = H$, $R^1 = OAc$, $R^2 = CH_2$ und $n = 1$.

11. Pharmazeutische Zusammensetzung nach Anspruch 1, dadurch gekennzeichnet, daß $R = H$, $R^1 = OAc$, $R^2 = \alpha$-Me(H) und $n = 1$.

12. Pharmazeutische Zusammensetzung nach Anspruch 1, dadurch gekennzeichnet, daß $R = H$, $R^1 = OAc$, $R^2 = \beta$-Me(H) und $n = 1$.

13. Pharmazeutische Zusammensetzung nach Anspruch 1, dadurch gekennzeichnet, daß $R = H$, $R^1 = OAc$, $R^2 = H_2$ und $n = 2$.

14. Pharmazeutische Zusammensetzung nach Anspruch 1, dadurch gekennzeichnet, daß $R = R^1 = H$, $R^2 = H_2$ und $n = 2$.

15. Pharmazeutische Zusammensetzung für die topische Anwendung auf die Haut eines Patienten, der an einer androgenbedingten Störung leidet, dadurch gekennzeichnet, daß sie aus einer 5α-Dihydrotestosteron herabsetzenden Menge einer Verbindung der Formel (I):

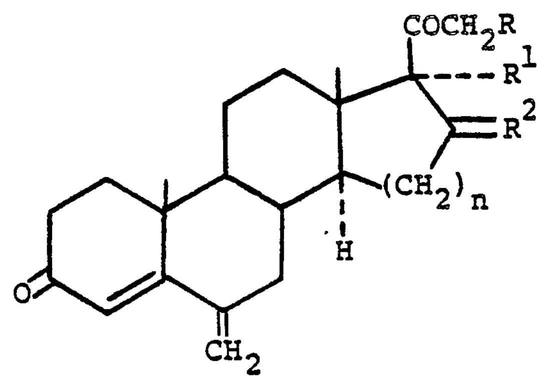

(I)

worin

R für H oder F steht;

$R^1$ aus —H; gerad- oder verzweigtkettigem niedrig Alkyl; Hydroxyl; $—OCOR^3$; und O—($C_1$—$C_6$-Alkyl); worin $R^3$ für H, eine $C_1$—$C_{10}$ gerad- oder verzweigtkettige Alkylgruppe, Phenyl, Phenylalkylen mit gerad- oder verzweigtkettigem $C_1$—$C_6$ Alkylen; $C_5$—$C_{10}$ Cycloalkyl oder $C_6$—$C_{10}$-Cycloalkylalkylen steht, ausgewählt ist;

$R^2$ für $H_2$, Methylen, Ethyliden, α-$CH_3CH$), β-$CH_3$(H), α-OH(H) oder für das vom 16α,17α-Dihydroxyderivat abgeleitete Acetonid steht und

n 1 oder 2 bedeutet, zusammen mit einem inerten pharmazeutisch zulässigen Träger besteht.

16. Zusammensetzung nach Anspruch 15, dadurch gekennzeichnet, daß der Träger aus ölartigen Grundlagen, Silikonen, Lanolinen, Polyethylenglykol, Glycerylmonostearat, Methylcellulose und Hydroxymethylcellulose ausgewählt ist.

**Revendications**

1. Composition pharmaceutique pour traiter les maladies liées aux androgènes chez l'homme, qui comprend des quantités décroissant en 5α-dihydrotestostérone d'un composé de formule (I):

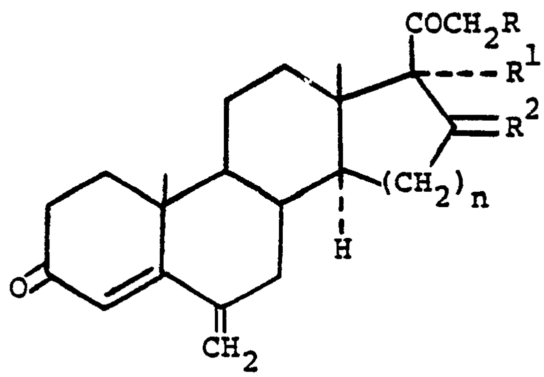

(I)

où

R représente H ou F;

$R^1$ est choisi parmi —H; alcoyle inférieur à chaîne droite ou ramifiée; hydroxyle; —$OCOR^3$; et O-(alcoyle en $C_1$ à $C_6$); où $R^3$ est —H, un groupe alcoyle à chaîne droite ou ramifée en $C_1$ à $C_{10}$, un phényle, phényl-alcoylène ayant un alcoylène en $C_1$ à $C_6$ à chaîne droite ou ramifiée; un cycloalcoyle en $C_5$ à $C_{10}$- ou cycloalcoyle en $C_6$ à $C_{10}$-alcoylène;

$R^2$ représente $H_2$, un méthylène, éthylidène, α-$CH_3$(H), β-$CH_3$(H), α-(OH)H ou l'acétonide provenant du dérivé 16α,17α-dihydroxy, et

n vaut 1 ou 2,

avec un support inerte pharmaceutiquement acceptable.

2. Composition pharmaceutique de la revendication 1 dans laquelle la maladie liée aux androgènes est choisie parmi l'acné, la séborrhée et l'alopécie androgénique.

3. Composition pharmaceutique de la revendication 2 dans laquelle le composé est administré sous la forme d'une préparation à usage local contenant de 0,001% à 5% de composé.

4. Composition pharmaceutique de la revendication 1 dans laquelle la maladie liée aux androgènes est choisie parmi la peau huileuse, l'hirsutisme, l'hypertrophie prostatique bénigne et l'adénocarcinome prostatique androgéno-dépendant.

5. Composition pharmaceutique de la revendication 4 dans laquelle le composé est administré par voie orale en une quantité allant de 0,1 à 50 mg/kg.

6. Composition pharmaceutique de la revendication 4 dans laquelle le composé est administré par voie parentérale en une quantité allant 0,1 à 50 mg/kg.

7. Composition pharmaceutique de la revendication 4 dans laquelle ladite maladie est l'adénocarcinome prostatique androgéno-dépendant et le composé est administré avec un composé choisi parmi l'acétate de mégestrol, la médrogestone et l'acétate de cyprotérone.

8. Composition pharmaceutique de la revendication 1 dans laquelle $R = R^1 = H$, $R^2 = H_2$ et $n = 1$.

9. Composition pharmaceutique de la revendication 1 dans laquelle $R = H$, $R^1 = OAc$, $R^2 = H_2$ et $n = 1$.

10. Composition pharmaceutique de la revendication 1 dans laquelle $R = H$, $R^1 = OAc$, $R^2 = CH_2$ et $n = 1$.

11. Composition pharmaceutique de la revendication 1 dans laquelle $R = H$, $R^1 = OAc$, $R^2$ représente α-Me(H) et $n = 1$.

12. Composition pharmaceutique de la revendication 1 dans laquelle $R = H$, $R^1 = OAc$, $R^2$ représente β-Me(H) et $n = 1$.

13. Composition pharmaceutique de la revendication 1 dans laquelle $R = H$, $R^1 = OAc$, $R^2 = H_2$ et $n = 2$.

14. Composition pharmaceutique de la revendication 1 dans laquelle $R = R^1 = H$, $R^2 = H_2$ et $n = 2$.

15. Composition pharmaceutique pour application locale à la peau d'un malade souffrant d'une maladie liée aux androgènes qui comprend des quantités dé-croissant en 5α-dihydrotestostérone d'un composé de formule:

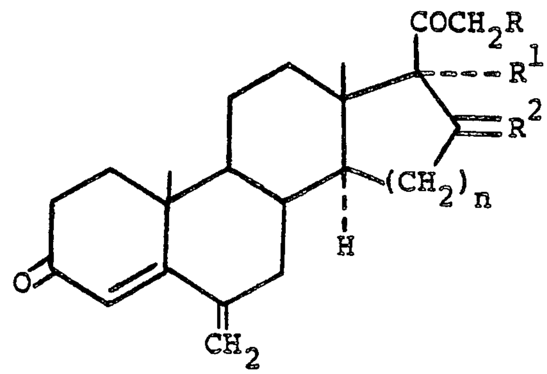

(I)

où

R représente H ou F;

$R^1$ est choisi parmi —H; alcoyle inférieur à chaîne droite ou ramifiée; hydroxyle; —$OCOR^3$ et O-(alcoyle en $C_1$ à $C_6$); où $R^3$ représente —H, un groupe alcoyle à chaîne droite ou ramifée en $C_1$ à $C_{10}$, phényle, phénylalcoylène ayant un groupe alcoyle a chaîne droite ou ramifiée en $C_1$ à $C_{10}$, phényle, phénylalcoylène ayant un alcoylène en $C_1$ à $C_6$ à chaîne droite ou ramifiée, cycloalcoyle en $C_5$ à $C_{10}$ ou cycloalcoyl-alcoylène en $C_6$ à $C_{10}$;

$R^2$ représente $H_2$, méthylène, éthylidène, α-$CH_3$(H), β-$CH_3$(H), α-(OH)H ou l'acétonide provenant du dérivé 16α,17α-dihydroxy, et

n vaut 1 ou 2;

avec un support pharmaceutique inerte à usage local.

16. Composition de la revendication 15 dans laquelle ledit support est choisi parmi les bases oléagineuses, les silicones, les lanolines, le polyéthylène-glycol, le monostéarate de glycéryle, la méthylcellulose et l'hydroxyméthylcellulose.

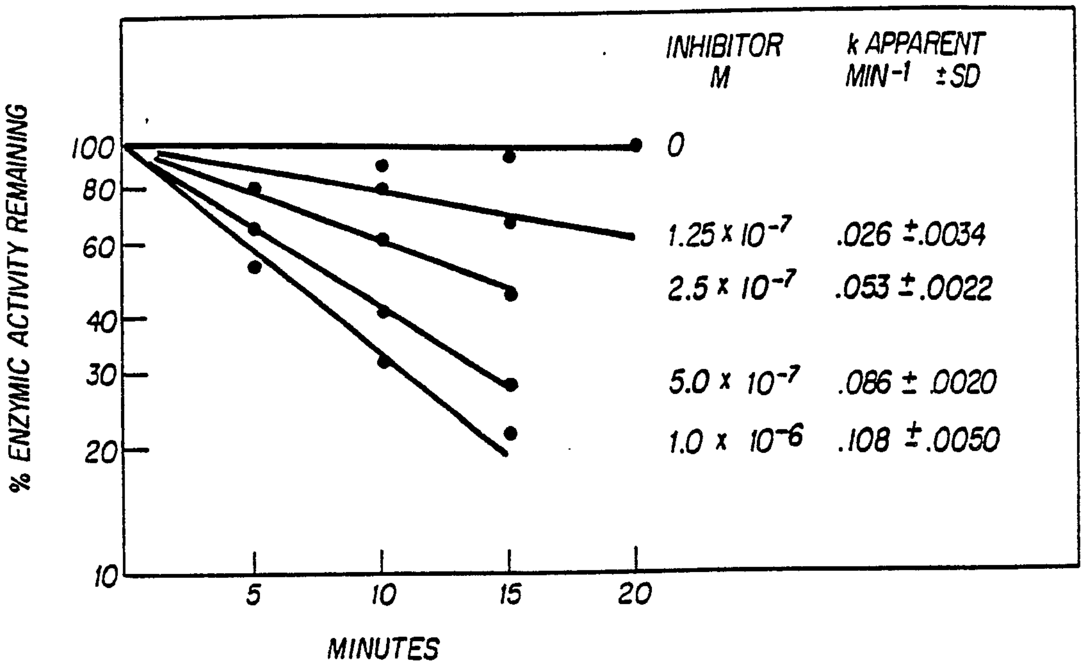
INHIBITOR
M
k APPARENT
MIN-1 ±SD
% ENZYMIC ACTIVITY REMAINING
100
80
60
40
30
20
10
0
1.25 x 10-7 .026 ±.0034
2.5 x 10-7 .053 ±.0022
5.0 x 10-7 .086 ± .0020
1.0 x 10-6 .108 ±.0050
5
10
15
20
MINUTES

FIG. 1

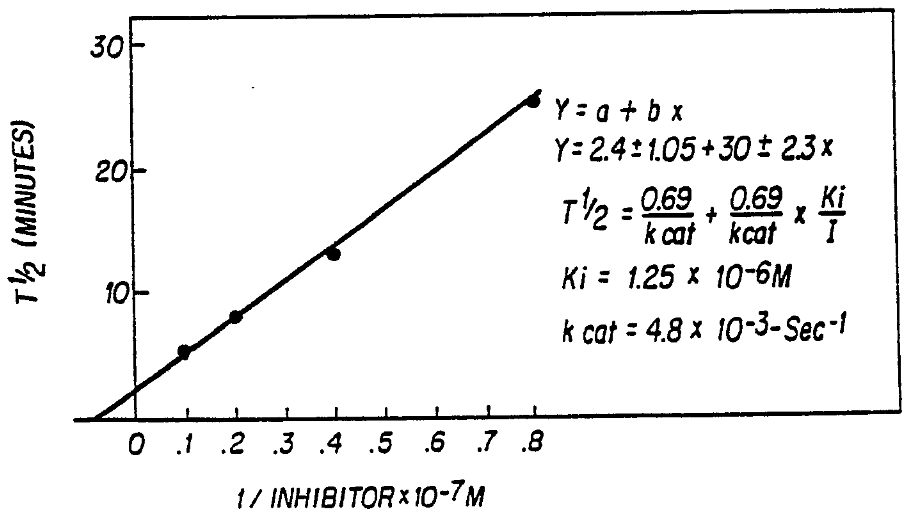
T½ (MINUTES)
30
20
10
Y = a + b x
Y= 2.4±1.05+30 ± 2.3 x
T½ = 0.69/k cat + 0.69/kcat x Ki/I
Ki = 1.25 x 10-6M
k cat = 4.8 x 10-3-Sec-1
0 .1 .2 .3 .4 .5 .6 .7 .8
1/ INHIBITOR x 10-7M

FIG. 2